# EUROPÄISCHE PATENTSCHRIFT

(11) **EP 1 362 584 B1**
(45) Veröffentlichungstag und Bekanntmachung des Hinweises auf die Patenterteilung: **04.02.2009**
(21) Anmeldenummer: 03014213.7
(22) Anmeldetag: 22.10.1997
(51) Int. Cl.: A61K 9/70

(54) **Sofortige Benetzbarkeit aufweisende(r) wasserlöslicher Film oder wasserlösliche Schicht zur oralen Applikation**
Immediate wettability water soluble film or water soluble layer for oral application
Film hydrosoluble ou couche hydrosoluble a mouillabilite immediate pour application orale

(30) Priorität: 11.11.1996 DE 19646392
(43) Veröffentlichungstag der Anmeldung: 19.11.2003
(62) Teilanmeldung aus: 97911237.2
(73) Patentinhaber: LTS LOHMANN Therapie-Systeme AG, 56626 Andernach (DE)
(72) Erfinder: Zerbe, Horst Georg, Hudson (Quebec) J0P1H0 (CA); Serino, Anthony J., Boonton, NJ 07005 (US); Guo, Jian-Hwa, Hudson, Ohio OH(330) 528,1461 (US)
(74) Vertreter: Flaccus, Rolf-Dieter

(56) Entgegenhaltungen:
- EP-A- 0 250 187
- EP-A- 0 355 536
- EP-A- 0 539 215
- WO-A-91/05540
- WO-A-96/25910
- DE-A- 2 432 925
- DE-A- 3 618 553
- US-A- 5 462 749

## Beschreibung

Die vorliegende Erfindung offenbart eine Zubereitung, die pharmazeutische Wirkstoffe und/oder die Atmung erfrischende Wirkstoffe enthalten, zur Anwendung in der Mundhöhle. Der Träger umfaßt wasserlösliche Polymere in Kombination mit bestimmten Inhaltsstoffen und hat eine therapeutische und/oder kosmetische Wirkung. Der Film wird unter Verwendung bestehender Beschichtungstechnologien aufgestrichen und getrocknet und weist eine sofortige Benetzbarkeit auf, gefolgt von schneller Auflösung/Zersetzung bei Applikation in der Mundhöhle.

An der Schleimhaut haftende (mukoadhäsive) Dosiersysteme zur Anwendung in der Mundhöhle, mit denen therapeutische und/oder kosmetische Wirkstoffe an die Mundschleimhaut abgegeben werden sollen, sind im Stand der Technik bekannt. Das US-Patent 5,047,244 beschreibt einen an der Mundschleimhaut haftenden Träger zur kontrollierten Abgabe eines therapeutischen Wirkstoffs durch das Schleimhautgewebe, der eine wasserfreie, aber hydratisierbare Polymermatrix und amorphes Siliciumdioxid enthält.

Fakultativ kann ein wasserunlöslicher Film beigefügt werden, um die Oberfläche nichtadhäsiv zu gestalten.

Die WO 91/06270 des gleichen Erfinders offenbart einen dreischichtigen Film zur verlängerten Abgabe eines aktiven Wirkstoffs in der Mundhöhle.

In gleicher Weise offenbart US 4,876,092 eine flächenförmige, adhäsive Zubereitung, umfassend eine adhäsive Schicht, welche bestimmte wasserlösliche und wasserunlösliche Polymere und einen wasserunlöslichen Träger enthält, welcher an der Mundschleimhaut haftet und dabei einen aktiven Wirkstoff an die Mundhöhle abgibt.

Alle diese vorgenannten Vorrichtungen sind nicht vollständig wasserlöslich und verbleiben in der Mundhöhle, selbst nach Erreichen des therapeutischen Ziels, und bereiten dem Patienten ein gewisses Unbehagen im Mund, das hauptsächlich durch die Trägerschicht verursacht wird, welche einen unlöslichen Rückstand im Mund zurückläßt.

Eine Reihe von Versuchen wurde unternommen, um das ungute Gefühl in der Mundhöhle zu verringern, welches durch die Starrheit und mangelnde Flexibilität der Trägerschicht verursacht wurde, indem man weiche Filmträger einführte. Die Dokumente EP 0 200 508 und EP 0 381 194 schlagen die Verwendung von Polyethylenfilmen, Polyvinylacetat, EthylenVinylacetat-Copolymeren, Metallfolien, Laminaten aus Stoff oder Papier und einem Plastikfilm, und ähnlichen Materialien als weiche Träger vor, wobei synthetische Harze wie Polyethylen, Vinylacetat-Homopolymere und Ethylen-VinylAcetat bevorzugte Materialien sind.

In gleicher Weise offenbart CA 1 263 312 die Verwendung von Polyolefinen wie Polyethylen, Polypropylen, Polyester, PVC, sowie Vliesstoffen als weiche Trägermaterialien.

Dennoch hinterlassen diese beim Patienten eine beträchtliche Menge von Rückständen des wasserunlöslichen Trägerfilms und verursachen daher immer noch ein unbehagliches Gefühl. Eine naheliegende Lösung zur Überwindung dieses Problems war die Entwicklung schleimhauthaftender Filme, welche vollständig zerfallen oder sich im Speichel auflösen.

Fuchs und Hilmann (DE 24 49 865. 5 und DE 24 32 925) stellten homogene, wasserlösliche Filme für buccale Zuführung von Hormonen her. Sie schlugen die Verwendung von wasserlöslichen Cellulosederivaten wie Hydroxyethylcellulose, Hydroxypropylcellulose oder Methylhydroxypropylcellulose als Filmbildner vor.

Die beiden Patentschriften DE 36 30 603 und EP 0 219 762 offenbaren die Verwendung von quellbaren Polymeren wie Gelatine oder Maisstärke als Filmbildner, welche nach Applikation in der Mundhöhle langsam zerfallen, wobei sie einen aktiven Wirkstoff freisetzen, der im Film enthalten ist. Die gleichen Polymere können ebenfalls verwendet werden, um Filme herzustellen, die zur Zahnpflege dienen sollen, entsprechend der Beschreibung in EP 0 452 446.

Aber auch diese Zubereitungen rufen ebenfalls ein ungutes Gefühl im Mund hervor, insbesondere verursacht durch ihre anfängliche Starrheit und verzögerte Erweichung. Hieraus resultiert ein Bedarf für eine Komposition zur Verwendung in der Mundhöhle, die dem Erfordernis eines Wohlgefühls im Mund Rechnung trägt.

Die vorliegende Erfindung offenbart Verfahren und zubereitungen, durch die ein unangenehmes Gefühl vermieden werden kann, und zwar durch Bereitstellen eines/einer sofortige Benetzbarkeit aufweisenden Films/Schicht zur Applikation an die Mundschleimhaut, und durch die eine Reißfestigkeit im freien Film erzielt wird, welche eine problemlose Beschichtun, Weiterverarbeitung und Verpackung eines verbraucherfreundlichen Produktes ermöglicht.

Die vorliegende Erfindung betrifft einen mucoadhäsiven, schnellauflösenden Film zur oralen Verabreichung, der an der Mundhöhle haftet und dabei einen pharmazeutischen oder kosmetischen Wirkstoff abgibt, wobei der Film mindestens ein wasserlösliches oder wasserdispergierbares Polymer, mindestens einen Polyalkohol und einen pharmazeutischen oder kosmetischen Wirkstoff, sowie eine Kombination von zwei oder mehreren nichtionischen oberflächenaktiven Stoffen enthält. Der Film enthält Hydroxypropylmethylcellulose als wasserlösliches oder wasserdispergierbares Polymer. Wahlweise kann die Formulierung eine Kombination von bestimmten Weichmacher, Farbstoffen, Süßmitteln, Geschmacksstoffen, Geschmacksverbesserern oder anderer Corrigenzien enthalten, wie sie üblicherweise zur Modifikation des Geschmacks von für die Applikation in der Mundhöhle bestimmten Formulierungen Verwendung finden. Der resultierende Film ist gekennzeichnet durch eine sofortige Benetzbarkeit, welche den Film unverzüglich nach Applikation an das Schleimhautgewebe erweichen läßt, wodurch beim Patienten ein länger anhaltendes unangenehmes Gefühl im Mund verhindert wird, sowie durch eine für normale Beschichtungs-, Schneid-, Aufschneid- und Verpackungsoperationen geeignete Reißfestigkeit.

Der mukoadhäsive Film der vorliegenden Erfindung enthält als wesentliche Bestandteile ein wasserlösliches Polymer oder eine Kombination von wasserlöslichen Polymeren, eine oder mehrere Weichmacher oder oberflächenaktive Stoffe, ein oder mehrere Polyalkohole und einen pharmazeutischen oder kosmetischen Wirkstoff.

Die für den mukoadhäsiven Film verwendeten Polymere umfassen hydrophile und/oder wasserdispergierbare Polymere. Bevorzugte Polymere sind wasserlösliche Cellulosederivate. Hydroxypropylmethylcellulose, Hydroxyethylcellulose und Hydroxypropylcellulose sind, allein oder gemischt, besonders bevorzugt. Beispiele für andere, fakultative, Polymere werden nachfolgend genannt, ohne die Erfindung einzuschränken: Polyvinylpyrrolidon, Carboxymethylcellulose, Polyvinylalkohol, Natriumalginat, Polyethylenglycol, natürliche Gummen wie Xanthanharz, Traganth, Guarharz, Akazienharz, Gummiarabicum, wasserdispergierbare Polyacrylate wie Polyacrylsäure, Methylmethacrylatcopolymer, Carboxyvinylcopolymere. Die Konzentration des wasserlöslichen Polymers im fertigen Film kann zwischen 20 und 75 Gew.-% betragen. Eine bevorzugte Konzentration beträgt zwischen 50 und 75 Gew.-%.

Die für den mukoadhäsiven Film verwendeten oberflächenaktiven Stoffe sind eine Kombination von zwei oder mehreren nichtionischen oberflächenaktiven Stoffen. Die erste Komponente kann ein Polyethoxysorbitan-fettsäureester oder ein α-hydro-ω-hydroxypoly(ethoxy)-poly-(propoxy)-poly(ethoxy)-Blockcopolymer sein, während die zweite Komponente ein Polyethoxyalkylether oder ein Polyethoxy-rizinusölderivat sein kann.

Bevorzugt soll der HLB-Wert der Polyethoxysorbitanfettsäurester zwischen 10 und 20 betragen, wobei ein Bereich zwischen 13 und 17 besonders bevorzugt ist. Das α-hydro-ω-hydroxypoly(ethoxy)-poly(propoxy)-poly(ethoxy)-Blockcopolymer soll mindestens 35 Propoxy-Einheiten, bevorzugt mindestens 50 Propoxy-Einheiten enthalten.

Der Polyethoxyalkylether sollte einen HLB-Wert zwischen 10 und 20 besitzen, bevorzugt nicht weniger als 15. Das Polyethoxy-rizinusölderivat soll einen HLB-Wert zwischen 14 und 16 besitzen.

Um die erwünschte, sofortige Benetzbarkeit zu erreichen, soll das Verhältnis zwischen der ersten und der zweiten Komponente einer binären oberflächenaktiven Mischung zwischen 1:10 und 1:1 gehalten werden, bevorzugt zwischen 1:5 und 1:3.

Die Gesamtkonzentration der oberflächenaktiven Stoffe im fertigen Film hängt ab von den Eigenschaften der anderen Ingredienzien, soll jedoch üblicherweise zwischen 0.1 und 5 Gew.-% betragen.

Der Polyalkohol wird benötigt, um den erwünschten Weichheitsgrad des Filmes zu erreichen. Beispiele von Polyalkoholen umfassen Glycerin, Polyethylenglycol, Propylenglycol, Glycerinmonoester mit Fettsäuren, oder sonstige pharmazeutisch verwendete Polyalkohole. Die Konzentration von Polyalkohol in der Trockenmasse des Filmes beträgt üblicherweise 0.1 bis 5 Gew.-%.

Der Film ist besonders gut geeignet zur Abgabe eines weiten Bereiches pharmazeutisch aktiver Wirkstoffe durch die Schleimhautmembranen eines Patienten, insbesondere durch die buccalen Schleimhäute.

Therapeutische Wirkstoffe, die Absorptionsprobleme infolge begrenzter Löslichkeit, Abbau im Gastrointestinaltrakt oder extensiven Metabolismus haben, sind besonders gut geeignet. Als Beispiele der einsetzbaren therapeutischen Wirkstoffe sind zu nennen: Hypnotica, Sedativa, Antiepileptica, Weckamine, Psychoneurotropica, Neuro-Muskelblocker, Antispasmodica, Antihistaminica, Antiallergica, Cardiotonica, Antiarrhythmica, Diuretica, Hypotensiva, Vasopressoren, Antitussiva, Expectorantia, Thyroidhormone, Sexualhormone, Antidiabetica, Antitumor-Wirkstoffe, Antibiotica sowie Chemotherapeutica und Narcotica. Die im Film einzulagernde Menge von Wirkstoff hängt von dessen Art ab und beträgt üblicherweise zwischen 0,01 und 20 Gew.-%, sie kann jedoch, falls zur Erzielung des gewünschten Effektes notwendig, höher liegen.

Kosmetische Wirkstoffe umfassen Atemerfrischer wie Menthol, andere Geschmacks-, Aroma- oder Duftstoffe, wie sie üblicherweise für die Mundhygiene verwendet werden, und/oder Wirkstoffe für Zahnpflege und/oder Mundhygiene, beispielsweise quartäre Ammoniumbasen. Die Wirkung von Geschmacks- und Aromastoffen kann durch Geschmacksverstärker wie Weinsäure, Zitronensäure, Vanillin oder dergleichen verstärkt werden.

Farbstoffe, welche wahlweise dem Film beigemischt werden, müssen hinsichtlich Toxizität sicher sein und sollten zur Verwendung in Kosmtetika durch die zuständigen Behörden zugelassen sein.

Der erfindungsgemäße mukoadhäsive Film kann folgendermaßen hergestellt werden:

Der Polyalkohol, oberflächenaktive Stoffe, Weichmacher und andere mögliche Bestandteile außer dem/den wasserlöslichen oder wasserdispersiblen Polymeren werden mit einer genügenden Menge eines kompatiblen Lösungsmittels gelöst. Beispiele eines kompatiblen Lösungsmittels umfassen Wasser, Alkohole oder deren Mischungen. Nach Bildung einer klaren Lösung wird das wasserdispersible Polymer oder die Mischung wasserdispersibler Polymere langsam unter Rühren und, falls notwendig, Hitze zugegeben, bis eine klare und homogene Lösung gebildet ist. Diese wird auf einen Träger aufgetragen und zu einem Film getrocknet. Das Trägermaterial muß eine Oberflächenspannung haben, die es ermöglicht, die Polymerlösung gleichmäßig über die vorgesehene Beschichtungsbreite zu verteilen, ohne daß die Lösung eingesaugt wird und somit eine destruktive Bindung zwischen Träger und Beschichtung entsteht.

Beispiele für geeignete Materialien umfassen nichtsilikonisierte Polyethylen-Terephthalat-Filme, nichtsiliconisiertes Kraftpapier, Polyethylen-imprägniertes Kraftpapier oder nichtsiliconisierten Polyethylenfilm.

Der Auftrag der Lösung auf das Trägermaterial kann mit jeder üblichen Vorrichtung ausgeführt werden. Eine speziell bevorzugte Auftragstechnik betrifft eine Walzenrakel-Streichmaschine.

Die Dicke der resultierenden Filmschicht hängt von der Konzentration der Feststoffe in der Beschichtungslösung sowie von der Spaltbreite der Beschichtungsmaschine ab und kann zwischen 5 und 200 um variieren. Die Trocknung des Films wird in einem Heißluftbad unter Verwendung eines Trokkenofens, Trockentunnels, Vacuumtrockners oder anderer geeigneter Trockenvorrichtungen vorgenommen, welche die Wirkung des/der Wirkstoffs/Wirkstoffe oder der Geschmacksstoffe nicht negativ beeinträchtigen. Um ein unangenehmes Gefühl im Mund zuverlässig zu vermeiden, soll die Filmdicke 70 µm nicht überschreiten. Zur besseren Gebrauchserleichterung kann der Film in Stücke von geeigneter Größe und Form geschnitten und verpackt werden.

Die Erfindung wird anhand nachfolgender Beispiele veranschaulicht.

### Beispiel 1:

15 g Sorbit, 6 g Glycerin, 0,5 g Polysorbat 80 (Tween 80), 2 g Brij 35, 25 g Zitronenminzearoma, 3 g Aspartam, 15 g 1-Menthol und 3 g Zitronensäure werden bei 60°C in einer Mixtur von 250 g Wasser und 250 g Ethanol solange gerührt, bis sich eine klare Lösung gebildet hat.

Zu der Lösung werden 30 g Hydroxypropylmethylcellulose langsam unter Rühren zugegeben, bis eine klare und homogene Lösung gebildet ist. Die resultierende Lösung wird dann bis auf Raumtemperatur abkühlen gelassen und unter Verwendung einer üblichen Beschichtungs-/Trocknungsvorrichtung auf ein geeignetes Trägermaterial aufgestrichen, beispielsweise nichtsiliconisiertes polyethylenbeschichtetes Kraftpapier. Beschichtungsspalt und Bahngeschwindigkeit müssen so geregelt werden, daß eine Trockenfilmdicke zwischen 20 und 50 µm erreicht wird. Die Trockentemperatur hängt von der Länge des Trockenofens und der Materialgeschwindigkeit ab und soll so eingestellt werden, daß die Lösungsmittel vollständig, oder zumindest weitgehend vollständig vom Film entfernt werden. Der resultierende Film wird vom Träger abgelöst und zum Gebrauch in Stücke von geeigneter Größe und Form zerteilt.

### Beispiel 2:

3 g Sorbit, 1,5 g Kollidon 30 (Lieferant: BASF), 5 g Glycerin, 5 g Propylenglycol, 5 g Polyethylenglycol, 4 g Polysorbat 80 (Tween 80), 8 g Brij 35, 12 g Pfefferminzaroma, 0,8 g Aspartam werden in einer Mischung von 400 g Wasser und 400 g Ethanol bei 60°C unter Rühren aufgelöst. Zu der klaren Lösung werden 28 g Hydroxypropylmethylcellulose unter Rühren langsam zugegeben. Nach völliger Auflösung des Polymers wird die Lösung auf Raumtemperatur abgekühlt und auf einen Träger unter den gleichen Beschichtungs- und TRocknungsbedingungen wie in Beispiel 1 aufgestrichen. Der trockene Film wird wieder in Stücke von geeigneter Größe und Form zerteilt.

### Beispiel 3:

15 g Sorbit, 22,5 g Glycerin, 2,5 Propylenglycol, 2,5 g Brij 35, 2,5 g Poloxamer 407, 3,5 g Cremophor RH 40, 9 g Kräuterminzearoma, 0,5 g Aspartam werden unter ständigem Rühren bei 60°C in einer Mischung von 250 g Wasser und 250 g Ethanol gelöst. Zu der klaren Lösung werden 75 g Hydroxypropylcellulose unter ständigem Rühren langsam zugegeben. Mit der klaren Lösung wird wiederum beschichtet und unter den beschriebenen Bedingungen getrocknet, wie in Beispiel 1; der trockene Film wird in Stücke von geeigneter Größe und Form zerteilt.

### Beispiel 4:

3,6 Tween 80, 3,6 g Glycerin, 39 g Menthol und 171 g Kollidon 30 werden bei Raumtemperatur und unter Rühren in einer Lösung aus 600 ml Wasser und 2800 ml Ethanol gelöst. 247,5 g Hydroxypropylmethylcellulose werden dann langsam und portionsweise bei 50-55 °C hinzugefügt; es wird bis zur vollständigen Auflösung gerührt. Nach Abkühlen der Mischung werden, in Folge, 90 g Zitronenminzearoma, gefolgt von einer Lösung/Suspension aus 27,13 g Aspartam, 18 g Zitronensäure und 0,17 g FD&C yellow #5 in 120 ml Wasser unter Rühren hinzugefügt. Die klare Lösung wird unter den in Beispiel 1 angegebenen Bedingungen aufgestrichen und getrocknet; der trockene Film wird in Stücke von für die beabsichtigte Verwendung geeigneter Größe und Form zerteilt.

## Patentansprüche

1. Mucoadhäsiver Film zur oralen Verabreichung, der mindestens ein wasserlösliches oder wasserdispergierbares Polymer, mindestens einen Polyalkohol und einen pharmazeutischen oder kosmetischen Wirkstoff enthält, **dadurch gekennzeichnet, daß** er Hydroxypropylmethylcellulose sowie eine Kombination von zwei oder mehreren nichtionischen oberflächenaktiven Stoffen enthält.

2. Mucoadhäsiver Film nach Anspruch 1, **dadurch gekennzeichnet, daß** die Gesamtkonzentration der oberflächenaktiven Stoffe 0,1 bis 5 Gew.-% beträgt.

3. Mucoadhäsiver Film nach Anspruch 1 oder 2, **dadurch gekennzeichnet, daß** die Kombination von nichtionischen oberflächenaktiven Stoffen als erste Komponente einen Polyethoxysorbitanfettsäureester oder ein α-hydro-ω-hydroxypoly(ethoxy)-poly(propoxy)-poly(ethoxy)-Blockcopolymer sowie als zweite Komponente einen Polyethoxyalkylether oder ein Polyethoxyrizinusölderivat enthält.

4. Mucoadhäsiver Film nach Anspruch 3, **dadurch gekennzeichnet, daß** der HLB-Wert der Polyelhoxysorbitanfettsäureester zwischen 10 und 20 beträgt, wobei ein Bereich zwischen 13 und 17 besonders bevorzugt ist.

5. Mucoadhäsiver Film nach Anspruch 3 oder 4, **dadurch gekennzeichnet, daß** der Polyethoxyalkylether einen HLB-Wert zwischen 10 und 20 besitzt, bevorzugt nicht weniger als 15.

6. Mucoadhäsiver Film nach einem der Ansprüche 3 bis 5, **dadurch gekennzeichnet, daß** das Polyethoxyrizinusölderivat einen HLB-Wert zwischen 14 und 16 besitzt.

7. Mucoadhäsiver Film nach einem der Ansprüche 3 bis 6, **dadurch gekennzeichnet, daß** das α-hydro-ω-hydroxypoly(ethoxy)-poly(propoxy)-poly(ethoxy)-Blockcopolymer mindestens 35, vorzugsweise mindestens 50 Propoxy-Einheiten enthält.

8. Mucoadhäsiver Film nach einem der Ansprüche 3 bis 7, **dadurch gekennzeichnet, daß** er eine binäre oberflächenaktive Mischung enthält, welche aus der genannten ersten und zweiten Komponente besteht, wobei das Verhältnis zwischen der ersten und der zweiten Komponente zwischen 1:10 und 1:1, vorzugsweise zwischen 1:5 und 1:3 gehalten wird.

9. Mucoadhäsiver Film nach einem der vorangehenden Anspruche, **dadurch gekennzeichnet, daß** die Konzentration von Polyalkohol in der Trockenmasse des Films 0,1 bis 5 Gew.-% beträgt.

10. Mucoadhäsiver Film nach einem der vorangehenden Ansprüche, **dadurch gekennzeichnet, daß** der/die Polyalkohol(e) aus der Glycerin, Polyethylenglykol, Propylenglykol, Glycerinmonoester mit Fettsäuren umfassenden Gruppe ausgewählt ist.

11. Mucoadhäsiver Film nach einem der vorangehenden Ansprüche, **dadurch gekennzeichnet, daß** die Konzentration des wasserlöslichen oder wasserdispergierbaren Polymers zwischen 20 und 75 Ges.-% beträgt.

12. Mucoadhäsiver Film nach einem der vorangehenden Ansprüche, **dadurch gekennzeichnet, daß** der Gehalt des im Film enthaltenen pharmazeutischen oder kosmetischen Wirkstoffs zwischen 0,01 und 20 Gew.-% liegt.

13. Mucoadhäsiver Film nach einem der vorangehenden Ansprüche, **dadurch gekennzeichnet, daß** er eine Dicke unterhalb von 70 µm besitzt.

14. Mucoadhäsiver Film nach einem der vorangehenden Ansprüche, **dadurch gekennzeichnet, daß** er einen therapeutischen Wirkstoff aus der Hypnotica, Sedativa, Antiepileptica, Weckamine, Psychoneurotropica, Neuro-Muskelblocker, Antispasmodica, Antihistaminica, Antiallergica, Cardiotonica, Antiarrhythmica, Diuretica, Hypotensiva, Vasopressoren, Antitussiva, Expectorantia, Thyroidhormone, Sexualhormone, Antidiabetica, Antitumor-Wirkstoffe, Antibiotica sowie Chemotherapeutica und Narcotica umfassenden Gruppe enthält.

15. Mucoadhäsiver Film nach einem der vorangehenden Ansprüche, **dadurch gekennzeichnet, daß** er einen kosmetischen Wirkstoff aus der Gruppe umfassend Atemerfrischer, Wirkstoffe für die Zahnpflege und Wirkstoffe für die Mundhygiene enthält.

16. Mucoadhäsiver Film nach einem der vorangehenden Ansprüche, **dadurch gekennzeichnet, daß** er Geschmacksstoffe, Aromastoffe oder Duftstoffe enthält.

17. Mucoadhäsiver Film nach einem der vorangehenden Ansprüche, **dadurch gekennzeichnet, daß** er 1-Menthol, Zitronenminzearoma, Pfefferminzaroma, Kräuterminzaroma, Aspartam und/oder Karamel enthält.

18. Verfahren zur Herstellung eines wasserlöslichen mucoadhäsiven Films nach einem der vorangehenden Ansprüche, umfassend die Schritte:
a) Lösen eines Polyalkohols, der nichtionischen oberflächenaktiven Stoffe, sowie aller anderer Bestandteile mit Ausnahme des/der wasserlöslichen oder wasserdispergierbaren Polymeren, in einem kompatiblen Lösungsmittel;
b) nach. Bildung einer klaren Lösung, Hinzugeben des/der genannten Polymers/Polymere unter Rühren bis zur Bildung einer Lösung;
c) Auftragen der Lösung auf ein Trägermaterial, welches eine Oberflächenspannung aufweist, die ein Einsaugen der Lösung verhindert;
d) Trocknen der Lösung unter Bildung eines Films.

## Claims

1. Mucoadhesive film for oral administration, containing at least one water-soluble or water-dispersible polymer, at least one polyalcohol and one pharmaceutical or cosmetic active agent, **characterised in that** it contains hydroxypropyl methylcellulose as well as a combination of two or more nonionic surfactants.

2. Mucoadhesive film according to claim 1, **characterised in that** the total concentration of the surfactants is 0.1 to 5%-wt.

3. Mucoadhesive film according to claim 1 or 2, **characterised in that** the combination of non-ionic surfactants contains, as a first component, a polyoxyethylene sorbitan fatty acid ester or a α-hydro-ω-hydroxypoly(oxyethylene)poly(oxypropylene)-poly(oxyethylene) block copolymer, and, as a second component, a polyoxyethylene alkyl ether or a polyoxyethylene castor oil derivative.

4. Mucoadhesive film according to claim 3, **characterized in that** the HLB value of the polyoxyethylene sorbitan fatty acid ester is between 10 and 20, with a range between 13 and 17 being particularly preferred.

5. Mucoadhesive film according to claim 3 or 4, **characterized in that** the HLB value of the polyoxyethylene alkyl ether is between 10 and 20, preferably not less than 15.

6. Mucoadhesive film according to claims 3 to 5, **characterized in that** the polyoxyethylene castor oil derivative has an HLB value between 14 and 16.

7. Mucoadhesive film according to any one of claims 3 to 6, **characterized in that** the α-hydro-ω-hydroxypoly-(oxyethylene)poly(oxypropylene)poly(oxyethylene) block copolymer contains at least 35, preferably at least 50, oxypropylene units.

8. Mucoadhesive film according to any one of claims 3 to 7, **characterized in that** it contains a binary surface-active mixture that consists of said first and second components, wherein the ratio between the first and second component is maintained between 1:10 and 1:1, preferably between 1:5 and 1:3.

9. Mucoadhesive film according to any one of the preceding claims, **characterized in that** the concentration of polyalcohol in the dry matter of the film is 0.1 to 5%-wt.

10. Mucoadhesive film according to any one of the preceding claims, **characterized in that** the polyalcohol(s) is/are selected from the group comprising glycerol, polyethylene glycol, propylene glycol and glycerol monoesters with fatty acids.

11. Mucoadhesive film according to any one of the preceding claims, **characterized in that** the concentration of the water-soluble or water-dispersible polymers is between 20 and 75%-wt.

12. Mucoadhesive film according to any one of the preceding claims, **characterized in that** the content of the pharmaceutically or cosmetically active agent contained in the film is between 0.01 and 20%-wt.

13. Mucoadhesive film according to any one of the preceding claims, **characterized in that** it has a thickness of less than 70 µm.

14. Mucoadhesive film according to any one of the preceding claims, **characterized in that** it contains a therapeutically active agent from the group comprising hypnotics, sedatives, antiepileptics, analeptic amines, psychoneurotropic agents, neuromuscular blocking agents, antispasmodic agents, antihistaminics, antiallergics, cardiotonics, antiarrhythmics, diuretics, hypotensives, vasopressors, antitussives, expectorants, thyroid hormones, sex hormones, antidiabetics, antitumor agents, antibiotics, as well as chemotherapeutics and narcotics.

15. Mucoadhesive film according to any one of the preceding claims, **characterized in that** it contains a cosmetically active agent from the group comprising breath freshening agents, active agents for dental care and active agents for oral hygiene.

16. Mucoadhesive film according to any one of the preceding claims, **characterized in that** it contains gustatory substances, flavouring substances or odoriferous substances.

17. Mucoadhesive film according to any one of the preceding claims, **characterized in that** it contains 1-menthol, Mentha citrata flavour, peppermint flavour, herb mint flavour, aspartame and/or caramel.

18. Process for the production of a water-soluble mucoadhesive film according to any one of the preceding claims, comprising the steps:
a) dissolving a polyalcohol, the nonionic surfactants as well as all other components, with the exception of the water-soluble or water-dispersible polymer(s), in a compatible solvent;
b) once a clear solution has formed, the above-mentioned polymer/polymers is/are added, with stirring, until a solution is formed;
c) applying the solution on a carrier material which has a surface tension that prevents absorption of the solution;
d) drying the solution, thereby forming a film.

## Revendications

1. Film mucoadhésif pour l'administration orale, qui contient au moins un polymère soluble dans l'eau ou apte à être dispersé dans l'eau, au moins un polyalcool et une substance active pharmaceutique ou cosmétique, **caractérisé en ce qu'**il contient de l'hydroxypropylméthylcellulose ainsi qu'une combinaison de deux substances tensioactives non ioniques ou plus.

2. Film mucoadhésif selon la revendication 1, **caractérisé en ce que** la concentration totale des substances tensioactives s'élève de 0,1 à 5 % en poids.

3. Film mucoadhésif selon la revendication 1 ou 2, **caractérisé en ce que** la combinaison des substances tensioactives non ioniques contient, à titre de premier composant, un ester d'acide gras de polyéthoxysorbitanne ou un copolymère séquencé α-hydro-(ω-hydroxypoly(éthoxy)-poly(propoxy)-poly(éthoxy) et, à titre de deuxième composant, un éther polyéthoxyalkylique ou un dérivé d'huile de ricin polyéthoxylée.

4. Film mucoadhésif selon la revendication 3, **caractérisé en ce que** la valeur HLB de l'ester d'acide gras de polyéthoxysorbitanne s'élève entre 10 et 20, une plage entre 13 et 17 étant particulièrement préférée.

5. _ Film mucoadhésif selon la revendication 3 ou 4, **caractérisé en ce que** l'éther polyéthoxyalkylique possède une valeur HLB entre 10 et 20, de préférence qui n'est pas inférieure à 15.

6. Film mucoadhésif selon l'une quelconque des revendications 3 à 5, **caractérisé en ce que** le dérivé d'huile de ricin polyéthoxylée possède une valeur HLB entre 14 et 16.

7. Film mucoadhésif selon l'une quelconque des revendications 3 à 6, **caractérisé en ce que** le copolymère séquencé a-hydro-m-hydroxypoly(éthoxy)-poly(propoxy)-poly(éthoxy) contient au moins 35, de préférence au moins 50 unités propoxy.

8. Film mucoadhésif selon l'une quelconque des revendications 3 à 7, **caractérisé en ce qu'**il contient un mélange tensioactif binaire qui est constitué par les premier et deuxième composants mentionnés, le rapport entre le premier et le deuxième composant étant maintenu entre 1:10 et 1:1, de préférence entre 1:5 et 1:3.

9. Film mucoadhésif selon l'une quelconque des revendications précédentes, **caractérisé en ce que** la concentration du polyalcool dans la matière sèche du film s'élève de 0,1 à 5 % en poids.

10. Film mucoadhésif selon l'une quelconque des revendications précédentes, **caractérisé en ce que** le/les polyalcool(s) est/sont choisi(s) parmi le groupe comprenant le glycérol, le polyéthylèneglycol, le propylèneglycol, des monoesters de glycérol avec des acides gras.

11. Film mucoadhésif selon l'une quelconque des revendications précédentes, **caractérisé en ce que** la concentration du polymère soluble dans l'eau ou apte à être dispersé dans l'eau s'élève entre 20 et 75 % en poids.

12. Film mucoadhésif selon l'une quelconque des revendications précédentes, **caractérisé en ce que** la teneur de la substance active pharmaceutique ou cosmétique contenue dans le film se situe entre 0,01 et 20 % en poids.

13. Film mucoadhésif selon l'une quelconque des revendications précédentes, **caractérisé en ce qu'**il possède une épaisseur inférieure à 70 µm.

14. Film mucoadhésif selon l'une quelconque des revendications précédentes, **caractérisé en ce qu'**il contient une substance active thérapeutique choisie parmi le groupe comprenant des hypnotiques, des sédatifs, des antiépileptiques, des weckamines, des neurotropes, des neurobloquants musculaires, des antispasmodiques, des antihistaminiques, des antiallergiques, des cardiotoniques, des antiarythmiques, des diurétiques, des hypotenseurs, des vasodépresseurs, des antitussifs, des expectorants, des hormones thyroïdiennes, des hormones sexuelles, des antidiabétiques, des substances actives antitumorales, des antibiotiques, ainsi que des agents chimiothérapeutiques et des narcotiques.

15. Film mucoadhésif selon l'une quelconque des revendications précédentes, **caractérisé en ce qu'**il contient une substance active cosmétique choisie parmi le groupe comprenant des agents rafraîchissant l'haleine, des substances actives pour les soins dentaires et des substances actives pour l'hygiène buccale.

16. Film mucoadhésif selon l'une quelconque des revendications précédentes, **caractérisé en ce qu'**il contient des agents de sapidité, des arômes ou des parfums.

17. Film mucoadhésif selon l'une quelconque des revendications précédentes, **caractérisé en ce qu'**il contient du 1-menthol, des arômes de menthe citronnée, des arômes de menthe poivrée, des herbes aromatiques au goût de menthe, l'aspartame et/ou du caramel.

18. Procédé pour la fabrication d'un film mucoadhésif soluble dans l'eau selon l'une quelconque des revendications précédentes, comprenant les étapes :
a) de dissolution d'un polyalcool, des substances tensioactives non ioniques ainsi que de tous les autres constituants, à l'exception du/des polymères solubles dans l'eau ou aptes à être dispersés dans l'eau, dans un solvant compatible ;
b) après la formation d'une solution claire, d'addition du/des polymères mentionnés, tout en agitant, jusqu'à l'obtention d'une solution ;
c) d'application de la solution sur une matière de support qui présente une tension superficielle qui empêche une pénétration de la solution par absorption ;
d) de séchage de la solution pour l'obtention d'un film.
